# EUROPEAN PATENT APPLICATION

(11) **EP 3 053 587 A1**
(43) Date of publication of application: **10.08.2016**
(21) Application number: 15153980.6
(22) Date of filing: 05.02.2015
(51) Int. Cl.: A61K 33/00, A61M 16/12, A61P 11/06, A61P 11/12

(54) **Combination of nitric oxide, helium and antibiotic to treat bacterial lung infections**

(71) Applicant: Linde AG, 80331 München (DE)
(72) Inventor: Jafri, Syed, London, W8 7HY (GB); Schmehl, Wolfgang, 82031 Grünwald (DE)
(74) Representative: Nordmeyer, Philipp Werner

(57) **Abstract**

The present invention relates to an apparatus (1) for the treatment of infections associated with respiratory disorders in a mammal with a mixture (2) for use as an inhalable medicament according to any of the preceding claims, comprising a patient interface (3), at least one source of helium for providing gaseous helium (21), at least one source of oxygen for providing gaseous oxygen (22), an application device for providing a mixture to a patient interface (4), at least one source of nitric oxide (23) for providing gaseous nitric oxide, a gas injector for injecting nitric oxide (5) provided by the source of nitric oxide (23) into the mixture (2) provided by the application device (4), an injector for injecting a means for inhibiting growth of pulmonary pathogens (6), a controller (8) programmed for controlling the gas injector (5), the application device (4) and the injector for injecting a means for inhibiting growth of pulmonary pathogens (6).

## Description

### Technical Field

The invention relates to a mixture as an inhalable medicament and a device for the application thereof to a patient.

### Technological Background

Patients with respiratory disorders often suffer from pulmonary luminal obstructions that impair breathing. The pathophysiological development of such disorders can cause fluid build-up in the lungs, leading to reduced gas exchange due to pulmonary shunting. In cases where the patient is unable to adequately clear these fluids, the concomitant obstructions in e.g. bronchioles and alveoli further reduces the efficacy of pulmonary gas exchange leading to insufficient blood oxygenation and blood acidification due to insufficient carbon dioxide expiration.

Such obstructions not only result in limited gas exchange but may also cause a coughing reflex or induce breathing spasms to improve clearance. However, for patients that are incapable to perform clearance, e.g. due to a reduced production of surfactant, in case of chronic diseases, or when the amount of fluid is too high for natural clearance, such pulmonary activation leads to irregular contractions. This further increases the patient's burden since this not only increase the patient's fatigue level and psychological distress, but also results in impaired pulmonary gas flow caused by unwanted vortexes and inadequate ventilation due to suboptimal breathing patterns. As a result, blood oxygenation is further reduced, leading to even more severe symptoms. This negative loop may in severe cases eventually lead to a collapse of the patient, requiring medical intervention.

The insufficient fluid clearance together with the altered pathophysiological conditions allows the intrusion of pulmonary pathogens, which leads to pulmonary infections such as pneumonia. Pneumonia is an inflammatory condition of the lung primarily affecting the alveoli resulting from infection with bacteria and/or viruses, less commonly by other organisms such as fungi or parasites. Bacteria generally enter the upper respiratory tract through aspiration of small quantities of microbial cells present in the nose or throat (particularly during sleep), via airborne droplets, or through gastric reflux. Systemic sepsis or septicaemia may also result in bacterial invasion of the lungs. Viral infection may occur through inhalation or distribution from the blood; in the lungs cells lining the airways, alveoli and parenchyma are damaged, and may render the patient more susceptible to bacterial infection of the respiratory tract.

In case of e.g. microbes such as bacteria, their rapid growth causes a change in the microenvironment, leading to acidification and cell death in pulmonary tissue. When pulmonary shunts exist this not only reduces the blood oxygenation, but also leads to a blood flow reduction. The normally occurring foreign body reaction is hence impaired since the inflammation induced mobilization and extravasation of host defence cells such as macrophages, leukocytes, natural killer cells, and/or mast cells to engulf and inactivate invading bacteria cannot sufficiently target the growth of pathogens. The reduced blood flow and clearance in addition lead to a build-up of necrotic and apoptotic cells, resulting in pus accumulation. Furthermore, the concomitant fluid extravasation into the alveoli from surrounding blood vessels may even worsen existing pulmonary shunts, further impairs breathing efficiency, restricts influx of respiratory gas to the affected alveoli thereby reducing gas exchange efficiency, and particularly damages the affected lower respiratory tract.

A further problem with bacterial growth is their secretion of macromolecules that provide an optimal microenvironment for proliferation. The accumulation of these macromolecules leads to the development of a so-called biofilm. Known to be a major cause for the gradual intolerance of medical implants, such biofilms are difficult to eliminate. The environment provided for these pathogens enables their colonization in a region that is difficult for the host's immune system to infiltrate or impairs induced extravasation, causing the readily formation of biofilms within 12 hours. Since bacteria preferably grow within the biofilm, known bactericidal agents such as antibiotics are mostly ineffective since they cannot penetrate and/or diffuse through the biofilm in sufficient concentrations to cause bacterial cell death. Ineffective clearance of such a biofilm may cause further accumulation and eventually leads to droplet formation and spreading of the pathogen through the respiratory system.

Reduced humidification, a reduced cough reflex and the impaired extravasation and infiltration of the immune system into the pathogenic area render the host's defense mechanism incapable of proper clearance. Prevention and treatment of these complications and symptoms are therefore of key interest for increasing survival.

Especially in the situation where pulmonary infections are associated with respiratory disorders, as described above, a systemic approach to target pulmonary infections, e.g. by ingestion and distribution through blood circulation of an antibiotic, would provide low efficiency since i) blood flow at the desired target site is often reduced due to shunting, ii) the delivery of the therapeutic is ineffective since the target site is not at a systemic localization, and iii) the low permeability of the biofilm does not provide sufficient diffusion of the therapeutic agent.

Hence, the problem with conventional therapies is that antibiotics are insufficiently directed to target sites and furthermore require high doses to provide a desired bactericidal effect. When bacterial growth is not properly targeted or the therapeutic effect is not readily achieved, this furthermore forms the risk of inducing bacterial resistance to these therapeutic agents, providing an even larger burden on the patient. These high doses in addition may cause unwanted and severe side effects, leading to e.g. ingestion and/or digestion problems, skin irritation and inflammation, dehydration, nausea etc.

For obvious reasons, opposed to e.g. dermatological disinfection, pulmonary infections cannot be treated through elimination with alcohol incubation. Simultaneously, a direct targeting through e.g. inhalation of an antibiotic is not possible in the current setting since i) this requires a corresponding medium for transportation which needs to be biocompatible, ii) existing obstructions may lead away from the targeting site, and iii) the antibiotic itself may form additional obstructions thereby further restricting air flow and gas exchange with an increase of concomitant health risks.

Other conventional methods that should prevent or reduce the risk of pneumonia or reduce the signs and symptoms thereof, such as frequent exogenous aspiration of secretions, prophylactic administration of antibiotics or saline lavage, in fact aggravate the patient's condition.

Alternative approaches include the application of nitric oxide and positive airway pressure or a combination thereof. Nitric oxide (NO) has many known biological functions. Ranging from neurotransmission, cellular differentiation to regulation of cellular oxygen consumption through effects on mitochondrial respiration, it also regulates the host immune response by e.g. inhibition of leukocyte adhesion and regulation of NFκB levels *in vivo.* Its use in treatment of diseases affecting the respiratory tract, however, bases on the relaxation of smooth muscle cells lining the vascular system.

Endogenously induced NO oxidizes the iron atom of a haem moiety in the enzyme soluble guanylate cyclase (SGC) in the smooth muscle cells of the lower respiratory tract airways, in the pulmonary arteries and in the membranes of circulatory platelets, thereby activating the SGC. The activated SGC forms the second messenger cGMP, which in smooth muscle cells promotes calcium-dependent relaxation, causing vasodilation of blood vessels in the lower respiratory tract, thereby increasing blood flow through the pulmonary arteries and capillaries, and also dilation of the airways in the lower respiratory tract, thereby improving bulk gas transport into the alveoli and exchange of O₂ and CO₂. Hence, administration of NO leads to a reduction of local blood pressure, stimulates vasodilatation and thereby facilitates gas exchange in the alveoli of the lungs. A further result is reduction of platelet aggregation on irregular surfaces (such as a constricted blood vessel) thereby lowering the probability of thrombosis (see WO 95 /10315 A1).

Biologically produced NO is synthesized *in vivo* by both constitutive and inducible isozymes of the nitric oxide synthases (NOS), which catabolize _{L}-arginine to NO and citrulline. Endothelial constitutive NOS (eNOS), present in the walls of bronchioles and pulmonary arterioles provide NO at nanomolar concentrations for regulating vessel tone.

Inhalable gaseous NO (IgNO) may be used to relax smooth muscle control of pulmonary arteriole diameter, for treating pulmonary hypertension in diseases such as acute respiratory distress syndrome (ARDS), in which impaired gas exchange and systemic release of inflammatory mediators ('acute phase proteins' and cytokines, particularly interleukins) cause fever and localised or systemic increases in blood pressure. IgNO will also relax smooth muscle control of bronchiole diameter, for treating emphysema in cases of ARDS and chronic obstructive pulmonary disease (COPD), in which the lower respiratory tract (particularly the lung parenchyma: alveoli and bronchioles) become inflamed. In COPD airways in the lower respiratory tract narrow and lung tissue breaks down, with associated loss of airflow and lung function which is not responsive to standard bronchodilating medication. IgNO administration may therefore assist in countering the 'pulmonary shunt', in which respiratory disease causes deregulation of the matching of the flow of air to the alveoli with the blood flow to the capillaries, which under normal conditions allows oxygen and carbon dioxide to diffuse evenly between blood and air (see WO 95 /10315 A1).

Furthermore, endogenously produced NO is partially responsible for the cytotoxic actions of macrophages due to its cell damaging activities. IgNO therefore has another advantage by providing an effective microbicidal molecule for treating infections of the respiratory tract that it acts directly *in situ,* whereas parenteral administration of drugs requires a high dosage to address systemic dilution and hepatic catabolism. Thus, NO has been shown to be an effective agent for killing *Mycobacterium tuberculosis* within cysts or tuberculi in a patient's lungs (see WO 00 / 30659 A1). IgNO may also be administered to treat pneumonia: pulmonary infection and inflammation (see WO 00 / 30659 A1).

Isozymes of inducible NOS (iNOS) are present in many cell types; upon activation they temporarily produce NO at micromolar concentrations, an activity which, under pathological conditions, has been associated with production of superoxides, peroxynitrites, e.g. upon reperfusion injury of ischemic tissue, inflammation and cellular damage. Hence, the application of NO has many negative side effects and can be highly toxic if applied in high dose for prolonged periods of time. The high reactivity of NO in pure form causes limited solubility in aqueous solutions. Consequently, delivery of NO is typically performed by administration of a prodrug which is metabolically degraded, or through direct inhalation of gaseous NO (IgNO).

The toxicity of IgNO is associated with a variety of properties.
(a) Firstly, NO is swiftly absorbed by lung tissue and enters the blood stream, where it reacts very rapidly with haemoglobin, oxidizing the iron atom of one of the four haem moieties to the ferric form, thereby creating stable methaemoglobin (+ nitrite and nitrate ions), inhibiting electron transport pathways and energy metabolism. Methaemoglobin's three ferrous haem groups have far greater affinity for oxygen than the haemoglobin haem moieties, so that blood in which the proportion of methaemoglobin is elevated releases insufficient oxygen to the tissues.
(b) Secondly, in the presence of oxygen NO reacts rapidly to form nitrogen dioxide (NO₂), itself a toxic molecule and forming acidic compounds in aqueous environments. Gaseous NO₂ at 5 ppm is considered to be a toxic concentration, compared to standard administrations of IgNO at between 10 to 40, maximum up to 80 ppm. As lung disease frequently causes reduced respiratory function, patients are often administered an O₂-enriched air supply. In the presence of such an increased concentration of O₂ the probability of NO being oxidized to toxic NO₂ is correspondingly greater.
(c) Thirdly, NO reacts with superoxides, increased in many disease states due to oxidative stress, to form toxic peroxynitrites, powerful oxidants capable of oxidizing lipoproteins and responsible, as are both NO and NO₂, for nitration of tyrosine residues. Peroxynitrite reacts nucleophilically with carbon dioxide, which is present at about 1 mM concentrations in physiological tissues, to form the nitrosoperoxycarbonate radical. This, in turn, degrades to form carbonate radical and NO₂, both of which are believed to be responsible for causing peroxynitrite-related cellular damage. Nitrotyrosine is used as an indicator of NO-dependent nitrative stress induced in many disease states, generally being absent or undetected in healthy subjects.
(d) Fourthly, NO₂ oxygenizes cobalt in cobalamin (vitamin B12), leading to a loss of serum methionine, responsible for the conversion of uridine to the nucleotide thymidine. The reduced availability leads to a loss of DNA production and/or repair and results in impaired cell division, cell-cycle arrest, and/or induced apoptosis.

Since, in the presence of oxygen, the NO concentration determines the production rate of NO₂, over-delivery of NO will generate excessive quantities of toxic NO₂. Even if inhaled for only a short period, excess NO may form sufficient methaemoglobin to reduce oxygen delivery to the tissues to dangerously low levels, particularly in patients suffering from lung disease. Excess inert carrier gas accompanying administration of IgNO may deplete oxygen content of respiratory gas supply. On the other hand, under-administration of IgNO to patients requiring relaxation of the smooth muscles in pulmonary arteries may result in excessively high arterial blood pressures causing a low partial pressure of O₂ in alveolar blood (low P_{A}O₂).

Consequently, precise control of the NO dosage is required at all times during administration of IgNO, in spite of irregular patient breathing patterns, fluctuations in ambient temperature and pressure, and depletion of the gas reservoir. The high reactivity with both the host (aqueous) environment and other gas molecules within a breathing gas, respiratory tract, and/or within circulation provides high risks with respect to *in vivo* toxicity. Furthermore, the poor targeting requires the application of a high NO concentration, which aggravates toxicity, especially where gas diffusion is impaired since this also prolongs its location specific retention time. Therefore lower NO concentrations are highly desirable, however, may no longer provide an effective therapeutic agent. Currently no therapy exists to effectively counter pulmonary infection related symptoms while warranting patient safety and/or reducing unwanted side effects to insignificant levels.

### Summary of the invention

It is an object of the present invention to provide a mixture for use as an inhalable medicament in the treatment of infections associated with respiratory disorders in a mammal, e.g. a human patient.

In a first aspect, a mixture for use as an inhalable medicament in the treatment of infections associated with respiratory disorders in a mammal is suggested, wherein the mixture is composed of a combination of at least helium, nitric oxide and oxygen, wherein the mixture further comprises a means for inhibiting growth of pulmonary pathogens.

The mixture according to the invention is to be understood to also include a gaseous mixture, even when certain components other than helium and oxygen are provided as a liquid (e.g. aerosol), a solid (e.g. in powder form), or a combination thereof. Bearing this in mind, the mixture could also be referred to as a "*gaseous mixture*".

The use of helium in this mixture as a carrier gas has many benefits. From technical diving, it is known that substitution of breathing gases by helium has many physiological effects and physiological benefits. For example, the pressure induced and accelerated tissue saturation with nitrogen can not only cause drowsiness and a delirious or narcotic state, potentially having neurotoxic effects, but also increases the risk of gaseous embolism formation. Furthermore, high oxygen saturation levels may become rapidly and increasingly neurotoxic upon crossing the blood-brain barrier. These potential life-threatening hazards are prevented by substituting significant concentrations of both nitrogen and oxygen by helium. In addition, the fact that helium is a chemically inert gas with high biocompatibility makes it an excellent carrier gas for medical applications.

Another advantage of using helium lies in the fact that it is highly dissolvable in blood, and has a large diffusibility. Together with its large affinity to oxygen it therefore provides improved oxygen transport, which reduces the burden of patients with respiratory disorders suffering from reduced blood oxygenation. The application of helium furthermore leads to a four to five times faster CO₂ diffusion, thereby significantly reducing hypercapnia and preventing acidosis due to pH normalization.

Furthermore, helium, being in the top most compartment of the periodic system, has a very low molecular mass and hence in gaseous form provides a very low density. In comparison with other gas molecules present in a breathing gas, such as nitrogen, oxygen and carbon dioxide, substitution of any of these molecules with helium according to the present invention hence provides an overall lower density of the mixture. This lower density consequently results in a lower Reynolds number, which is an indication for the flow pattern of a fluid and is a dimensionless quantity that is formed by dividing the density times speed times diameter by the viscosity of the fluid. In general, a Reynolds number of a fluid below 1000 can be considered to follow a laminar flow, while a Reynolds number above 2000 almost certainly provides a turbulent flow. During breathing a laminar flow, which is defined by parallel layers that do not laterally mix, is highly preferable to a turbulent flow, in which these layers are disrupted and the caused vortices can not only impair proper direction of the gas flow to the gas exchange site in the alveoli, but may also provide a differential accumulation of gas molecules, resulting in a concentration gradient and potential tissue incompatibility. The increased resistance accompanied with a turbulent flow furthermore requires an increased breathing effort and may further increase the burden of a patient. Hence, the lower Reynolds number provided with the substitution with helium provides improved breathing and gas exchange resulting in better blood oxygenation.

Since helium furthermore comprises a high expandability, this provides a lower drag of the gas flow and improves expansion of the alveoli, thereby alleviating potentially existing pulmonary shunts. In addition, this provides an optimal carrier gas for the transportation of other compounds in the mixture, such as nitric oxide, oxygen, or a therapeutic agent such as an antibiotic with no significant concentration loss by providing a flux. Since this simultaneously provides a better and more direct targeting of these compounds to the required site of infection, lower concentrations of these compounds can be used in order to be effective. Especially in the case of nitric oxide, and, albeit to a lesser extent, when choosing a therapeutic agent in form of an antibiotic, this greatly increases the safety of the patient by lowering the *in vivo* toxicity.

The pressure relief facilitated by the application of helium can have an analgesic effect by alleviating pain and apnoea sensation for patients while it does not increase safety issues since it does not havean anaesthetic effect such as other noble gases e.g. xenon. Its high thermal conductivity furthermore may provide a cooling effect which can have a palliative effect in cases of e.g. pulmonary acidosis. In addition, the above described expansion properties of helium facilitate breathing in several resting positions, which is especially beneficial for patients with comorbid conditions such as e.g. COPD, wherein seated positions are preferred for breathing and mucus clearance. Respiratory muscle fatigue or ventilatory failure can thus be reduced or even prevented.

The combination of helium with nitric oxide (NO) and oxygen leads to many synergistic advantageous effects. Not only does NO provide a mucolytic agent, i.e. having the capability to dissolve mucus to a limited extent, it also reduces the surface tension and viscosity thereof. Hence, the application of helium together with NO improves the targeting of the mixture to the desired site while improving the clearance of mucus, thereby further improving the gas flow and gas exchange.

Another advantage of using the above combination is provided by the NO induced increase of blood flow. As described above in more detail, NO specifically increases blood flow through the pulmonary arteries and capillaries through calcium-dependent relaxation of smooth muscle cells, and also dilation of the airways in the lower respiratory tract, thereby improving bulk gas transport into the alveoli and exchange of O₂ and CO₂. Hence, administration of NO leads to a reduction of local blood pressure, stimulates vasodilatation and thereby facilitates gas exchange in the alveoli of the lungs.

In another embodiment according to the invention, the means for inhibiting growth of pulmonary pathogens comprises an antibiotic, antimycotic, antiviral, and/or antiparasitic agent.

This synergistic therapeutic effect of increased clearance, reduced shunting, improved accessibility of alveoli, improved blood flow and increased blood oxygenation is of particular relevance for the application of a therapeutic agent such as an antibiotic. Not only does this improve the uptake and local distribution of the antibiotic through arterioles and capillaries to the surrounded affected inflammation sites, it furthermore facilitates the direct access of and targeting to the infected site.

Another advantage of using helium as a carrier gas is its biocompatibility and the fact that it is a chemically inert gas. Therefore, in another embodiment according to the invention, the means for inhibiting growth of pulmonary pathogens can be provided in an aerosol or powder form. The lack of reactivity and the reduced but yet effective concentrations of NO due to the combination with helium, as described above, allows this further addition without increasing toxicity or increasing the risk of introducing further pulmonary obstructions. This combination according to the invention leads to a highly synergistic and unexpected therapeutic effect.

Furthermore, NO not only has a bactericidal effect, as described above, but also has the capability of dispersing and reducing the formation of a biofilm. The altered surface tension and the accumulation of reactive oxygen and nitrogen species caused by NO drastically alters the microenvironment and growth conditions of a host's pathogen. In another aspect of the invention, the means for inhibiting growth of pulmonary pathogens can furthermore be capable of reducing the formation or dispersal of a biofilm. Again, this would synergistically increase the effectivity of both inhibiting the growth of pulmonary pathogens and reducing biofilm formation.

Thus, the combination of helium, nitric oxide, oxygen and a means for inhibiting growth of pulmonary pathogens, such as e.g. an antibiotic, as described above, has a highly synergistic therapeutic effect, thereby alleviating the patient's symptoms while reducing or at least not increasing the burden on a patient by providing the inhalable medicament. Due to its tolerance and its non-reactivity, further combinations can be incorporated. Therefore, in another aspect of the invention, the mixture can comprise another medicament for the treatment of a respiratory disorder or the treatment of complications thereof. The provided increased accessibility and blood flow result in a higher uptake and therapeutic effect of such medicaments.

The improved access to the respiratory system due to application of helium may further facilitate mechanical interventions such as e.g. laparoscopic removal, or other simultaneous therapies.

It has also been found that preferably said disorder to be treated could be selected from the group consisting of: a ventilator associated pneumonia (VAP), a toxoplasmosis, a heparin-protamine reaction, a traumatic injury, a traumatic injury to the respiratory tract, acidosis or sepsis, acute mountain sickness, acute pulmonary edema, acute pulmonary hypertension, acute pulmonary thromboembolism, adult respiratory distress syndrome, an acute pulmonary vasoconstriction, aspiration or inhalation injury or poisoning, asthma or status asthmaticus, bronchopulmonary dysplasia, hypoxia or chronic hypoxia, chronic pulmonary hypertension, chronic pulmonary thromboembolism, cystic fibrosis (CF), fat embolism of the lung, hyaline membrane disease, idiopathic or primary pulmonary hypertension, inflammation of the lung, perinatal aspiration syndrome, persistent pulmonary hypertension of a newborn, post cardiac surgery, a bacterial-, viral- and/or fungal bronchiolitis, a bacterial-, viral- and/or fungal pharyngitis and/or laryngotracheitis, a bacterial-, viral- and/or fungal pneumonia, a bacterial-, viral- and/or fungal sinusitis, a bacterial-, viral- and/or fungal upper and/or lower respiratory tract infection, a bacterial-, viral- and/or fungal- exacerbated asthma, a respiratory syncytial viral infection, bronchiectasis, bronchitis, chronic obstructive lung disease (COPD), cystic fibrosis (CF), acute respiratory distress syndrome (ARDS), emphysema, otitis, otitis media, primary ciliary dyskinesia (PCD), and pulmonary aspergillosis (ABPA) and cryptococcosis.

Preferably, the helium is present in the mixture in a concentration of between 30 to 80 percent, preferably between 50 and 75 percent, more preferred between 65 and 70 percent. Accordingly, the concentrations of the nitric oxide may be very low but the application thereof prevents or at least reduces the multiplication of bacteria, viruses and fungi which growth can be slowed down or even supressed. When higher concentrations of the ranges given above are used, bacteria, viruses and fungi can be killed.

Preferably, the oxygen is present in the mixture in a concentration of between 10 to 50 percent, preferably between 20 and 30 percent. Although blood oxygenation is often impaired in patients with respiratory disorders and hence inhaled oxygen concentrations are accordingly determined, the combination of e.g. helium and nitric oxide alleviate the patient's symptoms, which allows lower oxygen concentrations to be used. Not only does this further reduce potential reactivity with NO to produce toxic nitrous oxide, the lower oxygen concentration furthermore reduces the exposure to potential radical oxygen species which can have a detrimental and even neurotoxic effect.

The oxygen may be provided by using a source of pure oxygen such as a gas cylinder filled with pure oxygen.

The oxygen may also be provided using ambient air. Ambient air generally comprises oxygen levels of 20 to 21 percent and furthermore comprises high levels of nitrogen, a low level of carbon dioxide and negligible levels of other elements. Ambient air can thus be used to provide the mixture with a sufficient oxygen level. If higher oxygen doses are desired the mixture can furthermore be enriched by oxygen. This has e.g. the advantage that lower amounts of enriched or pure oxygen are required, reducing the costs and logistical effort of the application of the mixture. As described above, the combination of helium with ambient air may furthermore increase the bactericidal effect.

Obviously, air can also be used to supplement the mixture to facilitate e.g. helium and/or oxygen levels in the lower limit or if e.g. a high level of either helium or oxygen is desired and the total mixture does not reach 100 percent.

The source of helium and the source of oxygen can be kept separated. However, the source for helium and oxygen can also be a common source such as a reservoir containing a helium/oxygen gaseous mixture. Such mixtures are known in the art as Heliox, examples of which can be found in e.g. WO 2010049078 or US 20030077330. However, a combination of helium and air as a common source, or a separate source for helium and air, may also be provided.

Preferably, the nitric oxide is present in the mixture in a concentration of between 10 ppm and 1000 ppm, preferably between 10 ppm and 250 ppm, more preferred between 10 ppm and 20 ppm or between 80 ppm and 160 ppm or between 160 ppm and 250 ppm. Accordingly, the concentrations of the nitric oxide may be very low but a continuous application thereof prevents or at least reduces the multiplication of bacteria, viruses and fungi by slowing down or even supressing growth. When higher concentrations of the ranges given above are used, bacteria, viruses and fungi can be killed, biofilm formation can be reduced and/or prevented, and blood flow and oxygenation is improved.

The mucolytic and pulmonary blood pressure reducing properties of NO together with the microbicidal effect have an unexpected synergistic effect. While acute treatment to increase oxygen exchange in the alveoli uses high concentrations (i.e. up to maximal 1000ppm or 250 ppm) of NO, continuous long-term application with such levels is impossible due to associated high toxicity.

The use of low doses is compliant with clinical levels and falls within the exposure levels for the approved indications. As is the case with neonatal infants, such doses can be applied for a prolonged time of >14 days, a time period normally exceeding the required time for reducing growth of pulmonary pathogens.

As a source of nitric oxide, nitric oxide can also be produced at the required location, i.e. in-line. Such production methods are known in the art. US 5,396,882, WO 2013/052548 A2, and WO 2014/143842 A1 disclose e.g. the application of an electric arc or plasma to enrich NO from air, whereas US 2006/172018 provides a method for obtaining NO by controlling the diffusion and/or dissolution of nitride salts or other precursor compositions. NO can also be derived through a reaction with reduction agents. An example of such a method can e.g. be found in US 2011/220103. The methods described here should not be appreciated such that these are limiting, but merely provide examples from a plurality of alternative methods known in the art.

It is another aspect of the invention to provide an apparatus for the treatment of infections associated with respiratory disorders in a mammal, e.g. a patient, with a mixture for use as an inhalable medicament. Such an apparatus comprises a patient interface and an application device for providing a mixture to a patient interface. In order to provide gaseous helium and oxygen, it comprises at least one source of helium, at least one source of oxygen, and furthermore comprises at least one source of nitric oxide for providing gaseous nitric oxide. For injecting nitric oxide provided by the source of nitric oxide into the mixture provided by the application device, the apparatus furthermore comprises a gas injector. To inject a means for inhibiting growth of pulmonary pathogens, the apparatus comprises a further injector. Furthermore, the apparatus comprises a controller programmed for controlling the gas injector, the application device and the injector for injecting a means for inhibiting growth of pulmonary pathogens.

In a further embodiment according to the invention, the source of oxygen is replaced or supplemented by a source of air. This may be ambient air or e.g. a source of compressed air. It may further be desired and/or required to implement air filters when using ambient air to prevent potential infiltration of e.g. aerosol pathogens present in the ambient air into the respiratory system.

Furthermore, in another embodiment according to the invention, the nitric oxide may be provided and generated in-line. For example, NO may then be mixed into the mixture at a gas injector and/or may replace a nitric oxide source. Such methods and apparatuses are known in the field and allow the generation of NO through nitrogen and oxygen present in ambient air by for example a pulsating electrical discharge or an electric arc, see e.g. WO 2013/05248 A2 and WO 2014/143842 A1, respectively.

According to the invention, the controller can further be programmed to control the application device to provide the mixture at adjustable intervals and for adjustable periods of time.

This is especially beneficial for patients and therapies, wherein the gas inhalation is performed over prolonged periods of time and frequently needs to be interrupted for e.g. further therapeutic treatment, personal hygiene measures, or nourishment. It can also be that the application of high concentrations of e.g. nitric oxide and/or e.g. an antibiotic are preferred for treatment and the corresponding increase of toxicity and the patient's burden is reduced by choosing respective application intervals and application times. During periods in which no mixture is applied, the patient can then breathe ambient air.

The continuous application of e.g. antibiotics prevents an accidental (temporary) therapy stop and hence reduces the concomitant risk of developing antibiotic resistance. As described above, the lower concentration but very effective targeting due to synergistic composition of the mixture, together with the continuous application, increases the efficacy of the therapy and reduces the patients' burden.

In another aspect of the invention, the controller can be programmed to control the application device to provide the mixture at predetermined phases during exhalation and inhalation. Especially in case of spontaneous breathing, the application of the mixture is preferred to occur during the inhalation phase. Dependent on the patient specific disease, conditions and symptoms, the mixture may e.g. be provided at the onset of the inhalation phase to facilitate the breathing initiation due to the presence of helium, and/or may be provided at a later phase of inhalation to further increase the helium facilitated expansion of alveoli and to provide the inhalable medicament in deeper regions of the respiratory tract. The patient interface and the application device can be configured to allow breathing of ambient air during phases where no application of the mixture or occurs or allow simultaneous breathing of ambient air.

Although the application according to exhalation and inhalation phases may occur e.g. at predetermined breathing intervals, it is preferred that the mixture is provided dependent on the actual breathing phase. Therefore, the apparatus may furthermore comprise a flow rate sensor to sense the flow rate of the mixture provided to the patient interface.

The controller can thereby be furthermore programmed to control the application device to provide the mixture dependent on the flow rate measured by the flow rate sensor. As such, the specific breathing phase can be determined according to the measured flow rate and the application of the mixture can be performed accordingly. The application can also occur with brief pulses at different frequencies during a specific breathing phase and/or a specific breathing interval. For example, the mixture is supplied to the breathing gas in at least a pulse throughout an inhalation cycle, during every second breathing cycle or during every other natural number of breathing cycles except one. When supplying the mixture in pulses, higher concentrations of e.g. nitric oxide and/or an antibiotic may be used, in particular concentrations towards the upper end of the ranges given above. The measured flow rate can furthermore provide the controller with information to adjust the provision of the mixture, the injection of nitric oxide and/or the injection of a means for inhibiting growth of a pulmonary pathogen.

To balance potential remaining negative effects of the mixture, e.g. of nitric oxide or an antibiotic, it is also contemplated to supply the mixture during an interval of between 1 minute to 60 minutes, preferably between 1 minute and 30 minutes, more preferably between 5 minutes and 20 minutes with an intermission between two subsequent intervals of between 1 minute to 1 day, preferably of between 1 hour and 8 hours, more preferably between 2 hours and 6 hours, most preferred between 3 hours and 5 hours. In other words, treatments with the mixture are carried out intermittently and preferably with higher concentrations of nitric oxide and/or e.g. an antibiotic but in the intermissions the organism can recover again.

To find a balance between the positive and negative characteristics of nitric oxide, the nitric oxide is preferably injected into the breathing gas of the positive airway pressure device during an interval of between 1 minute and 60 minutes, preferably between 15 minutes and 30 minutes, with an interval every second day up to 5 intervals a day.

Furthermore, when using intervals with intermissions in between it is also possible to synchronize the intermissions with the growth rates of the bacteria, viruses and fungi to be suppressed. In other words, this administration regime aims at killing the bacteria, viruses and fungi at a certain point in time by administering e.g. a certain dose of nitric oxide and/or a means for inhibiting the growth of a pulmonary pathogen - either as a pulse or a number of subsequent pulses or as a continuous treatment during a treatment interval. Then the supply of the mixture is shut off. Remaining bacteria, viruses and fungi may then start multiplying again but are killed again before they have reached a critical concentration by administering the next interval or pulse/pulses of the mixture. Accordingly, the duration of the intermission depends on the growth rate of the bacteria, viruses and fungi determined as critical.

Hence, the concentration levels of the different compounds in the mixture, the supply time, and the supply type can be chosen both patient and therapy dependently. Furthermore, the concentrations and therapy time can be varied while the desired total volume of the applied mixture is remained constant to achieve maximum efficacy.

Preferably, the controller is programmed to provide the continuous application of the mixture, but only the injection of the nitric oxide and/or the means for inhibiting the growth of a pulmonary pathogen occurs according to the above intervals, pulses, and/or periods of time. For example, this could mean that the controller is programmed to continuously apply the mixture and controls the gas injector to inject the nitric oxide every second breathing cycle or every other natural number of breathing cycles except one. Accordingly, the nitric oxide is added to the breathing gas not every breathing cycle, i.e. every inhalation phase, but every other or even less frequently.

In order to provide to the patient a suitable concentration of nitric oxide which can be administered to specific areas of the lung, the controller is programmed to control the gas injector to inject the nitric oxide preferably in a single pulse, in multiple subsequent pulses of equal or varying width, or as a constant flow with a varying or fixed flow rate such that different areas of the lung can be reached by the nitric oxide. By triggering the different spikes of the injection of nitric oxide, the depth within the lungs that can be reached by the nitric oxide can be varied. The different pulses may have differing widths, depending on the respective prescription.

Another advantage of the present invention is that the use of helium can have a neuroprotective effect (WO 2008122655). Its high expandability can however be further effective when combined with positive airway pressure. Not only does this provide a further relief and reduces effort for patients having breathing difficulties, it can also provide gas flow to regions in the respiratory tract that are difficult to access using spontaneous breathing only. Such an application may furthermore be provided at different intervals and/or with different pulses, as described above.

Accordingly, in another embodiment, the apparatus according to the invention comprises a positive airway pressure device to provide the mixture at an adjustable pressure. Here, the controller is programmed to control the positive airway pressure device. Dependent on the configuration, this positive airway pressure device may replace the application device.

Precise application of the mixture can be achieved by the provision of a gas sensor for sensing and analyzing the gas in the patient interface, preferably a gas sensor for determining the concentration of helium, nitric oxide, and/or oxygen , wherein the gas sensor is in communication with the controller. Accordingly, the apparatus comprises a gas sensor upstream of the patient interface to measure the concentrations of the gases to be inhaled in the mixture and which is in communication with the controller to accordingly adjust the injection of nitric oxide. Alternatively, the gas sensor may also be provided at or within the patient interface.

Similarly, in order to measure the exhaled gas concentrations, gas exchange and efficacy of the applied mixture, a gas sensor to sense the gases during an exhaling phase can be provided. Accordingly, the apparatus according to the present invention comprises a gas sensor downstream of or within the patient interface to measure the exhaled gas concentrations and which is in communication with the controller to accordingly adjust the injection of nitric oxide.

Preferably, the injector for injecting a means for inhibiting growth of pulmonary pathogens comprises a nebulizer. Such a nebulizer can either be in direct communication with the injector or can be provided as an autonomous injector that replaces said injector. The injection may be provided continuously, e.g. in very low doses, but preferably occurs at predetermined intervals and with predetermined frequencies and/or pulses, as described above.

Alternatively, the injector for injecting a means for inhibiting growth of pulmonary pathogens comprises a dry powder inhaler. Such an inhaler can likewise be either in direct communication with the injector or can be provided as an autonomous injector that replaces said injector. The injection may be provided continuously, e.g. in very low doses, but preferably occurs at predetermined intervals and with predetermined frequencies and/or pulses, as described above.

It is a further aspect of the invention to provide an apparatus, wherein the application device is configured to provide the mixture under mechanical ventilation. In such an embodiment, the nitric oxide and means for inhibiting growth of a pulmonary pathogen are injected into a flow of the mixture which is provided under positive pressure by an invasive mechanical ventilator and the nitric oxide is injected together with a carrier gas such that the gas injected is already in the correct concentration. The enriched mixture is then provided to the patient interface.

The invasive mechanical ventilator senses the pressure in the airways and automatically induces a positive pressure and a positive airflow when a lower pressure threshold is achieved, thereby providing a positive airflow to the patient interface, and stops the provision of a positive airflow when an upper threshold is achieved, allowing passive efflux of air during a passive exhaling phase until the lower threshold is again achieved.

In order to meet the prescriptions, the controller is preferably programmed to control the injection of the nitric oxide and the means for inhibiting the growth of a pulmonary pathogen into the breathing gas of the invasive mechanical ventilator during autonomous mechanical ventilation and during supportive mechanical ventilation during continuous mechanical ventilation and is duration-independent.

By the same token, it is preferred that the controller is programmed to interrupt the gas injector to provide nitric oxide and the injector to inject a means for inhibiting the growth of a pulmonary pathogen when the invasive mechanical ventilator detects an exhaling phase.

According to the invention the elements of the apparatus can be configured such that it is transportable. It is to be understood that this could mean that the apparatus can be either completely mobile, i.e. portable or moveable by e.g. a patient or medical trained personnel, or at least provide elements that can be detachably arranged. For example, instead of using central gas supplies through wall connectors, offered in many medical facilities, small volume gas tanks can be provided instead to provide a mobile apparatus with portable and exchangeable elements.

The apparatus according to the invention may further contemplate that all components mediating gas flow are biocompatible and are inert with the mixture. Especially for application to a patient's interface this is of essential importance since any occurring gas reaction from a gas source towards the patient interface may produce toxic compounds that are detrimental effects and reduce the patient's safety. For example, all applied tubing are provided from a biocompatible materials and dissolvable coatings that may release and/or expose reactive oxides should be avoided.

In addition to the nitric oxide injection and the injection of a means for inhibiting the growth of a pulmonary pathogen, it is a further aspect of the invention that the apparatus comprises means to provide another medicament for the treatment of a respiratory disorder or the treatment of complications thereof to the mixture. Due to the high tolerance and the non-reactivity of the mixture and the relevant components of the apparatus, these further combinations can be incorporated. The provided increased accessibility and blood flow result in a higher uptake and therapeutic effect of such medicaments. The provision of another medicament can be performed by the same injector that injects the means for inhibiting growth of a pathogen by provision of e.g. a second reservoir and/or a mixing and dispensing chamber, or may also be performed by a separate injector. The injector can furthermore be configured to comprise a nebulizer and/or a dry powder inhaler as described above.

In another embodiment according to the present invention, the apparatus comprises means to filter, collect, and/or transfer exhaled breathing gases to prevent dissipation of the mixture into ambient air. This not only reduces potential contamination of exhaled air with the to be inhaled mixture, which may provide unwanted gas concentrations to be inhaled and/or an inefficient respiration, but also provides increased safety for surrounding patients, medical personnel and the environment.

The exhaled breathing gases can furthermore be used to measure concentrations of e.g. metabolic waste products such as carrier compounds, nitrogen, ammonia, or lactates. These measurements may provide information about e.g. drug absorption and/or metabolism. Hence, according to another embodiment of the present invention, the apparatus comprises means to measure molecular concentrations present in the exhaled breathing gas. Such means are known in the art, such as e.g. chemical sensors.

According to another embodiment of the present invention, the apparatus comprises means to measure flow characteristics of the exhaled breathing gas. Flow characteristics may be measured to provide information such as e.g. breathing resistance, turbulence, or breathing volume, which can provide an indication of potential obstructions and/or the efficacy of the therapy. Such means are also known in the art, such as e.g. flowrate sensors.

The above means for measuring molecular concentrations present in the exhaled breathing gas and the means to measure flow characteristics of the exhaled breathing gas may be combined in a single means, such as e.g. a single sensor. Furthermore, the apparatus may comprise a separate controller or a control unit within a common controller for processing the acquired data. As such, the therapy may be adjusted accordingly.

Furthermore, the apparatus may comprise a safety means, in case the acquired data exceed predetermined thresholds. Such a safety means may e.g. comprise an emergency stop of the application or transmission of an emergency signal, when e.g. concentrations measured in the exhaled breathing gas indicate the occurrence of complications. For example, if a patient stops breathing for a certain period of time or the volume or consistency of the exhaled breathing gas are distinct from predefined values, e.g. indicate increased acidosis, an alarm may be triggered and/or the level of e.g. oxygen may be adjusted accordingly.

### Brief description of the drawings

The present disclosure will be more readily appreciated by reference to the following detailed description when being considered in connection with the accompanying drawings in which:
Figure 1 is a schematic view of an apparatus for providing a mixture to a patient;
Figure 2 is a schematic view of another apparatus for providing a mixture to a patient. In the following, the invention will be explained in more detail with reference to the accompanying Figures. In the Figures, like elements are denoted by identical reference numerals and repeated description thereof may be omitted in order to avoid redundancies.

### Detailed description of preferred embodiments

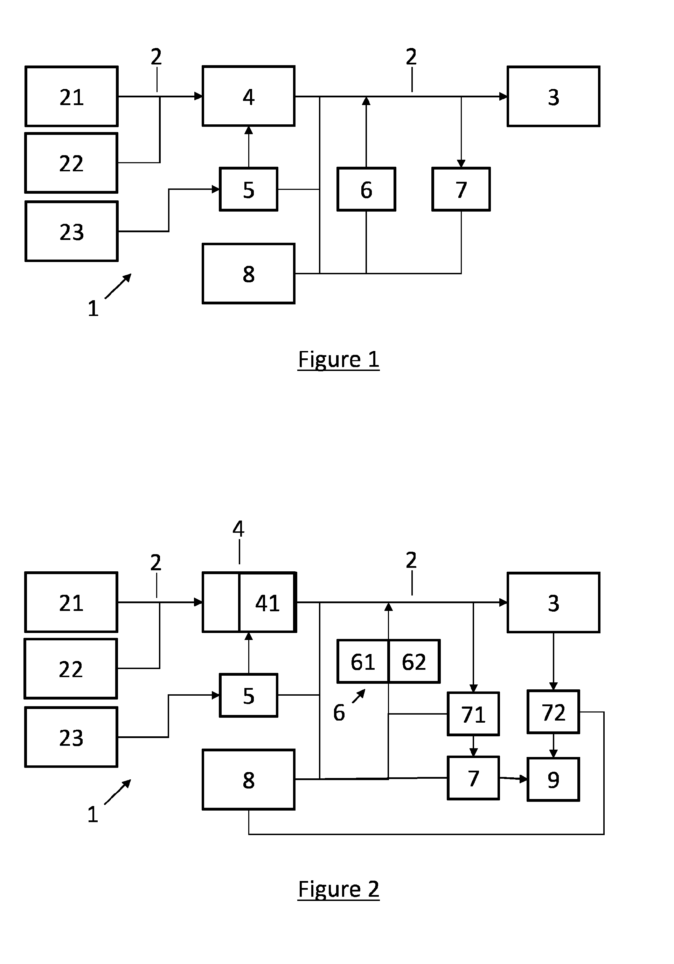

In Figure 1 an apparatus for providing a mixture to a patient is shown, as indicated by 1. A mixture 2 is provided to an application device and originates from a source of helium 21 and a source of oxygen 22. The gases can also be provided as a mixture of helium and oxygen, also called heliox, which is known in the art and can be stored in e.g. a container or tank. An application device 4 can be any device for the delivery of a mixture as known in the art.

The source of oxygen may be a source of pure oxygen such as a gas cylinder filled with pure oxygen. The source of oxygen may also be a source of air (not shown). This may be ambient air or e.g. a source of compressed air. It may further be desired and/or required to implement air filters when using ambient air to prevent potential infiltration of e.g. aerosol pathogens present in the ambient air into the respiratory system (not shown).

Nitric oxide coming from a nitric oxide source 23 is injected into the mixture 2 by a gas injector 5. Although Figure 1 shows that the injection is performed at or in the application device 4, the nitric oxide injection may also occur at a point in the mixture 2 before or after the application device 4 if configured accordingly.

The nitric oxide may be provided and generated in-line by e.g. implementing a subunit that generates NO by employing an electric arc and using both nitrogen and oxygen from ambient air. For example, NO may then be mixed into the mixture at a gas injector 5 and/or may replace a nitric oxide source 23 (not shown).

A means for inhibiting growth of a pulmonary pathogen is injected into the mixture 2 by an injector 6. Again, this injection may occur at a point in the mixture 2 after the application device 4, but this may also occur before or in the application device and/or before or after the injection of nitric oxide into the mixture 2.

The mixture 2 enriched with nitric oxide and a means for inhibiting the growth of a pulmonary pathogen, such as an antibiotic, is then provided at the downstream patient interface 3 to be inhaled as a medicament by the patient.

Furthermore, the apparatus 1 is provided with a controller 8 to control the injection of the gas injector 5 and the injector for injecting of a means for inhibiting growth of a pulmonary pathogen 6. In addition, the controller 8 controls the application device 4 for the provision of the mixture 2 and can do so depending on gas flow measurements provided by the flow rate sensor 7. For example, upon initiation of an inhalation phase by the patient, gas flow through the patient interface 3 can cause a pressure decrease and a flow rate to be measured by the flow rate sensor 7. When the controller 8 receives this information it can control the injection of nitric oxide and e.g. an antibiotic while providing the mixture 2 by actuating the application device 4.

By the same token, an exhalation phase may cause a pressure increase through the patient interface 3, which causes the flow rate sensor 7 to detect an exhalation phase. As soon as the exhalation phase is terminated, the corresponding loss of pressure may also provide the controller 8 with measurements that cause the application device 4 to provide the mixture 2, with or without further injection of nitric oxide or a means for inhibiting growth of a pulmonary pathogen. In doing so, the provision of the mixture 2 may occur before the inhalation phase is initiated by the patient, which further reduces the breathing effort and facilitates an improvement in pulmonary gas influx.

The apparatus may further comprise an in-line heating or cooling unit to provide the mixture at a desired and/or patient specific temperature.

In Figure 2 another exemplary embodiment of an apparatus for providing a mixture to a patient according to the present invention is shown, as indicated by 1. In addition to the apparatus 1 from Figure 1, a feedback mechanism is provided to ensure that the appropriate gas concentrations are provided at the patient interface 3.

The feedback mechanism comprises of at least an inhaled gas sensor 71 and an exhaled gas sensor 72. After injection of nitric oxide and/or a means for inhibiting growth of a pulmonary pathogen, the mixture 2 is mediated across an inhaled gas sensor 71. This sensor preferably measures the concentration of nitric oxide, but alternatively can also measure the concentration of helium and oxygen in the mixture 2. Preferably, as shown in Figure 2, the inhaled gas sensor 71 is in line with the flow rate sensor 7 to both minimize the size of the apparatus 1 and to reduce the amount of gas lost. However, other embodiments are possible, wherein these measurements are performed in parallel. The measured values are processed by the controller 8, which is in communication with the sensor 7, 71 and which can accordingly adjust the injection of nitric oxide, the means for inhibiting growth of a pulmonary pathogen and/or the total provision of the mixture 2. The excess gas is then led to an exhaled gas container and/or filter 9 to store and or filter the mixture and prevent leakage to the environment and/or ambient air.

Another feedback mechanism is comprised by the exhaled gas sensor 72. Preferably exhaled gas from the patient interface 3 is led through the exhaled gas sensor 72 before being led further to the exhaled gas container and/or filter 9. The exhaled gas sensor 72 is in communication with the controller 8, which can accordingly adjust the injection of nitric oxide, the means for inhibiting growth of a pulmonary pathogen and/or the total provision of the mixture 2 to adjust the medical treatment and/or prevent the unwanted *in vivo* accumulation of potentially toxic compounds.

According to the embodiment in Figure 2, the application device can furthermore comprise a positive airway pressure device 41 to apply the mixture 2 under positive pressure. This can be an advantage in cases where spontaneous breathing is insufficient and/or where lower regions of the respiratory tract, which would otherwise would not sufficiently be ventilated, require increased gas flow and/or gas exchange.

Furthermore, also according to the embodiment in Figure 2, the apparatus 1 allows the injection of a second medicament through an injector for injecting a second medicament 62. Although Figure 2 shows that this injection occurs after the injection of nitric oxide and downstream of the application device 41, it may also be possible that this occurs simultaneously at the application device 4 or upstream thereof.

In addition, the injector for injecting a means for inhibiting growth of a pulmonary pathogen may comprise a nebulizer 61 for provision of a medicament in aerosol form, or may even be replaced by a nebulizer if accordingly configured.

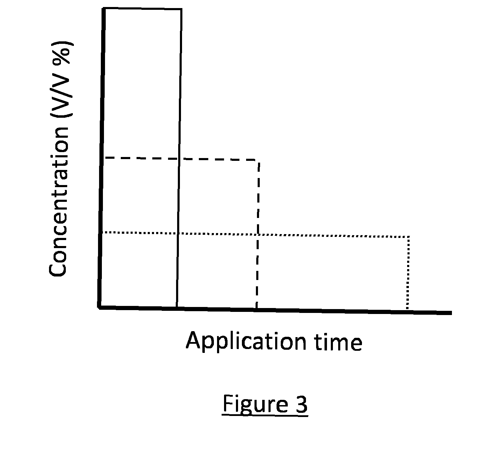

Figure 3 shows an example of different concentrations and time intervals that can be used for providing the mixture to a patient. When using high concentrations (vertical axis), the time of the application (horizontal axis) can be accordingly reduced (compare solid line, dashed line and dotted line). The total volume of the mixture that is provided to the patient (total area derived by concentration times application time), however, remains the same. The concentration and application time hence form a reciprocal correlation.

Further embodiments of the apparatus can be summarized as disclosed in the following items:
1. Apparatus 1 for the treatment of infections associated with respiratory disorders in a mammal with a mixture 2 for use as an inhalable medicament, comprising a patient interface 3,
   at least one source of helium for providing gaseous helium 21,
   at least one source of oxygen for providing gaseous oxygen 22,
   an application device for providing a mixture to a patient interface 4,
   at least one source of nitric oxide 23 for providing gaseous nitric oxide,
   a gas injector for injecting nitric oxide 5 provided by the source of nitric oxide 23 into the mixture 2 provided by the application device 4,
   an injector for injecting a means for inhibiting growth of pulmonary pathogens 6,
   a controller 8 programmed for controlling the gas injector 5, the application device 4 and the injector for injecting a means for inhibiting growth of pulmonary pathogens 6.
2. Apparatus 1 according to 1, wherein the at least one source of oxygen for providing gaseous oxygen 22 comprises a source of ambient air.
3. Apparatus 1 according to claim 1 or 2, wherein the at least one source of nitric oxide 23 is a source for in-line production and provision of nitric oxide.
4. Apparatus 1 according to any of 1 to 3, wherein the controller 8 is programmed to control the application device 4 to provide the mixture 2 at adjustable intervals and for adjustable periods of time.
5. Apparatus 1 according to any of 1 to 4, wherein the controller 8 is programmed to control the application device 4 to provide the mixture 2 at predetermined phases during exhalation and inhalation.
6. Apparatus 1 according to any of 1 to 5, wherein it comprises a flow rate sensor 7 for sensing the flow rate of the mixture 2 provided to the patient interface 3.
7. Apparatus 1 according to 6, wherein the controller 8 is programmed to control the application device 4 to provide the mixture 2 dependent on the flow rate measured by the flow rate sensor 7.
8. Apparatus 1 according to any of 1 to 7, wherein it comprises a positive airway pressure device 41, wherein the controller 8 is programmed to control the positive airway pressure device 41 to provide the mixture 2 at an adjustable pressure.
9. Apparatus 1 according to any of 1 to 8, wherein it comprises a gas sensor 71 upstream of the patient interface 3 to measure the concentrations of the gases to be inhaled in the mixture 2 and which is in communication with the controller 8 to accordingly adjust the injection of nitric oxide.
10. Apparatus 1 according to any of 1 to 9, wherein it comprises a gas sensor 72 downstream of or within the patient interface 3 to measure the exhaled gas concentrations and which is in communication with the controller 8 to accordingly adjust the injection of nitric oxide.
11. Apparatus 1 according to any of 1 to 10, wherein the injector for injecting a means for inhibiting growth of pulmonary pathogens 6 comprises a nebulizer 61.
12. Apparatus 1 according to any of 1 to 11, wherein the injector for injecting a means for inhibiting growth of pulmonary pathogens 6 comprises a dry powder inhaler.
13. Apparatus 1 according to any of 1 to 12, wherein the application device 4 is configured to provide the mixture 2 under mechanical ventilation.
14. Apparatus 1 according to any of 1 to 13, wherein its elements are configured such that it is transportable.
15. Apparatus 1 according to any of 1 to 14, wherein all components mediating gas flow are biocompatible and are chemically inert with the mixture 2.
16. Apparatus 1 according to any of 1 to 15, wherein it comprises means to provide another medicament 62 for the treatment of a respiratory disorder or the treatment of complications thereof to the mixture 2.
17. Apparatus 1 according to any of 1 to 16, wherein it comprises means to filter, collect, and/or transfer exhaled breathing gases 9 to prevent dissipation of the mixture into ambient air.

It will be obvious for a person skilled in the art that these embodiments and items only depict examples of a plurality of possibilities. Hence, the embodiments shown here should not be understood to form a limitation of these features and configurations. Any possible combination and configuration of the described features can be chosen according to the scope of the invention.

### List of reference numerals

- 1: Apparatus for the treatment of infections associated with respiratory disorders
- 2: Mixture
- 21: Source of helium
- 22: Source of oxygen
- 23: Source of nitric oxide
- 3: Patient interface
- 4: Application device
- 41: Positive airway pressure device
- 5: Gas injector for injecting nitric oxide
- 6: Injector for injecting a means for inhibiting growth of pulmonary pathogens
- 61: Nebulizer
- 62: Injector for injecting a second medicament
- 7: Flow rate sensor
- 71: Inhaled gas sensor
- 72: Exhaled gas sensor
- 8: Controller
- 9: Exhaled gas container and/or filter

## Claims

1. Mixture (2) for use as an inhalable medicament in the treatment of infections associated with respiratory disorders in a mammal, wherein the mixture (2) is composed of a combination of at least helium, nitric oxide and oxygen, **characterized in that** the mixture (2) furthermore comprises a means for inhibiting growth of pulmonary pathogens.

2. Mixture (2) according to claim 1, **characterized in that** the means for inhibiting growth of pulmonary pathogens comprises an antibiotic, antimycotic, antiviral, and/or antiparasitic agent.

3. Mixture (2) according to claim 1 or 2, **characterized in that** the means for inhibiting growth of pulmonary pathogens is provided in an aerosol or powder form.

4. Mixture (2) according to any of the preceding claims, **characterized in that** it furthermore comprises another medicament for the treatment of a respiratory disorder or the treatment of complications thereof.

5. Mixture (2) according to any of the preceding claims, **characterized in that** a respiratory disorder can be a ventilator associated pneumonia (VAP), a toxoplasmosis, a heparin-protamine reaction, a traumatic injury, a traumatic injury to the respiratory tract, acidosis or sepsis, acute mountain sickness, acute pulmonary edema, acute pulmonary hypertension, acute pulmonary thromboembolism, adult respiratory distress syndrome, an acute pulmonary vasoconstriction, aspiration or inhalation injury or poisoning, asthma or status asthmaticus, bronchopulmonary dysplasia, hypoxia or chronic hypoxia, chronic pulmonary hypertension, chronic pulmonary thromboembolism, cystic fibrosis (CF), fat embolism of the lung, haline membrane disease, idiopathic or primary pulmonary hypertension, inflammation of the lung, perinatal aspiration syndrome, persistent pulmonary hypertension of a newborn, post cardiac surgery, a bacterial-, viral- and/or fungal bronchiolitis, a bacterial-, viral- and/or fungal pharyngitis and/or laryngotracheitis, a bacterial-, viral- and/or fungal pneumonia, a bacterial-, viral- and/or fungal sinusitis, a bacterial-, viral- and/or fungal upper and/or lower respiratory tract infection, a bacterial-, viral- and/or fungal-exacerbated asthma, a respiratory syncytial viral infection, bronchiectasis, bronchitis, chronic obstructive lung disease (COPD), cystic fibrosis (CF), acute respiratory distress syndrome (ARDS), emphysema, otitis, otitis media, primary ciliary dyskinesia (PCD), and pulmonary aspergillosis (ABPA) and cryptococcosis.

6. Mixture (2) according to any of the preceding claims, **characterized in that** it comprises a helium concentration of between 30 and 80 percent, preferably between 50 and 75 percent, more preferably between 65 and 70 percent.

7. Mixture (2) according to any of the preceding claims, **characterized in that** it comprises an oxygen concentration of between 10 and 50 percent, preferably between 20 and 30 percent.

8. Mixture (2) according to any of the preceding claims, **characterized in that** it comprises a nitric oxide concentration of between 10 ppm and 1000 ppm, preferably between 10 ppm and 250 ppm, more preferred between 10 ppm and 20 ppm or between 80 ppm and 160 ppm or between 160 ppm and 250 ppm.

9. Apparatus (1) for the treatment of infections associated with respiratory disorders in a mammal with a mixture (2) for use as an inhalable medicament according to any of the preceding claims, comprising
a patient interface (3),
at least one source of helium for providing gaseous helium (21),
at least one source of oxygen for providing gaseous oxygen (22),
an application device for providing a mixture to a patient interface (4),
at least one source of nitric oxide (23) for providing gaseous nitric oxide,
a gas injector for injecting nitric oxide (5) provided by the source of nitric oxide (23) into the mixture (2) provided by the application device (4),
an injector for injecting a means for inhibiting growth of pulmonary pathogens (6),
a controller (8) programmed for controlling the gas injector (5), the application device (4) and the injector for injecting a means for inhibiting growth of pulmonary pathogens (6).

10. Apparatus (1) according to claim 9, **characterized in that** the at least one source of oxygen for providing gaseous oxygen (22) comprises a source of ambient air.

11. Apparatus (1) according to claim 9 or 10, **characterized in that** the at least one source of nitric oxide (23) is a source for in-line production and provision of nitric oxide.

12. Apparatus (1) according to any of the claims 9 to 11, **characterized in that** the controller (8) is programmed to control the application device (4) to provide the mixture (2) at adjustable intervals and for adjustable periods of time.

13. Apparatus (1) according to any of the claims 9 to 12, **characterized in that** the controller (8) is programmed to control the application device (4) to provide the mixture (2) at predetermined phases during exhalation and inhalation.

14. Apparatus (1) according to any of the claims 9 to 13, **characterized in that** it comprises a flow rate sensor (7) for sensing the flow rate of the mixture (2) provided to the patient interface (3).
